Europäisches Patentamt

European Patent Office

Office européen des brevets

(11) Publication number: **0 121 013**
**B1**

# EUROPEAN PATENT SPECIFICATION

(45) Date of publication of patent specification: **13.05.87**

(51) Int. Cl.⁴: **A 61 K 7/18**, A 61 K 33/16

(21) Application number: **83301833.6**

(22) Date of filing: **31.03.83**

(54) **Oral compositions.**

(43) Date of publication of application:
**10.10.84 Bulletin 84/41**

(45) Publication of the grant of the patent:
**13.05.87 Bulletin 87/20**

(84) Designated Contracting States:
**AT BE CH DE FR GB IT LI NL SE**

(56) References cited:
**GB-A- 644 339**

**JOURNAL OF THE AMERICAN CHEMICAL
SOCIETY, vol. 80, 5th June 1958, pages 2662-
2664, H.M. HAENDLER et al.: "The synthesis of
ammonium fluorometallates in methanol"**

**JOURNAL OF THE TEXTILE INSTITUTE, vol. 70,
no. 7, July 1979, pages 308-315, P.A. CUSACK
et al.: "A study of flame-resist treatments of
wool by inorganic tin chemicals"**

**CHEMICAL ABSTRACTS, vol. 94, 1981, page 32,
no. 95768j, Columbus, Ohio, USA, N.
TINANOFF et al.: "Microbiological,
ultrastructural and spectroscopic analyses of
the anti-tooth-plaque properties of fluoride
compounds in vitro"**

(73) Proprietor: **UNILEVER PLC
Unilever House Blackfriars P.O. Box 68
London EC4P 4BQ (GB)**
(84) **GB**

(73) Proprietor: **UNILEVER NV
Burgemeester s'Jacobplein 1 P.O. Box 760
NL-3000 DK Rotterdam (NL)**
(84) **BE CH DE FR IT LI NL SE AT**

(72) Inventor: **Viccaro, John Peter
145-77 7th Avenue
Whitestone New York (US)**

(74) Representative: **Doucy, Robert Henry et al
Unilever PLC Patents Division P.O. Box 68
Unilever House
London EC4P 4BQ (GB)**

Courier Press, Leamington Spa, England.

## Description

This invention relates to compositions for the care of the oral cavity.

The efficacy of fluorine in the prevention of dental caries is well established (see for example US Patents 3 029 191, 3 070 510 and 3 227 617). The acceptance of fluoride ions as an anticariogenic agent is based essentially on the observation that among the many topical agents that have been tested in clinical trials only those that contain fluorine have been effective in reducing caries. Fluorine-free salts tested have not prevented caries formation.

It is known that sodium hexafluorostannate ($Na_2SnF_6$) applied topically to the teeth has a caries inhibiting effect (Caries Res. 3, 315—325, 1969).

We have now found that stannic ammonium fluoride, $Sn(NH_4)_2F_6$, (which may also be called ammonium hexafluorostannate), as well as having an anti-caries effect also has a plaque inhibiting effect (which has not been reported for sodium hexafluorostannate) and furthermore that stannic ammonium hexafluorostannate has an important practical advantage over the use of sodium hexafluorostannate in that it has a significantly better taste than the sodium compound.

Accordingly the invention provides an oral composition having anti-caries and anti-plaque activity comprising 0.01 to 3% by weight of stannic ammonium fluoride [$Sn(NH_4)_2F_6$].

The synthesis of $Sn(NH_4)_2F_6$ is described in J.Am.Chem.Soc. *80*, 2662, 1958.

In the preparation of therapeutic or prophylactic oral compositions incorporating $Sn(NH_4)_2F_6$, a suitable carrier or oral media well known in the art is used. Adjuvants such as colouring agents, flavours, humectants, abrasives, detergents, preservatives, emollients and the like and other therapeutic agents compatible with $Sn(NH_4)_2F_6$ may also be included.

The oral composition is preferably in the form of a toothpaste or mouthwash. A particularly suitable amount of the $Sn(NH_4)_2F_6$ is such as to provide from 1000 to 1500 ppm of fluoride ion. However, the amount may conveniently range from 100 to 3000 ppm.

The following Experiments and Examples illustrate the invention. Percentages are by weight.

The $Sn(NH_4)_2F_6$ employed in the following experiments was synthesised according to the method of Haendler and Johnson (J.Am.Chem.Sec. *80*, 2662, 1958). The analytical details were as follows:

|        | Theoretical % | Analytical % |
|--------|---------------|--------------|
| Sn     | 44.18         | 45.64        |
| $NH_4$ | 13.41         | 12.65        |
| F      | 42.41         | 43.14        |

Cariogenic Bacteria used for testing the effect of $Sn(NH_4)_2F_6$ were *Streptococcus mutans* (strains coded 6715 and OMZ-176), *Streptococcus sanguis* (strains coded 10556 and 10557), *Streptococcus salivarius* (strains coded SS4 and H257), and *Lactobacillus casei.*

All of the above organisms were maintained as lyophilised cultures until the time for use.

An aqueous solution of $Sn(NH_4)_2F_6$ has a pH of about 3.8 as compared to a pH of 3.0 for $SnF_2$. This higher pH characteristic eliminates some of the formulation problems encountered with the strong acid effect of $SnF_2$. The preferred pH of oral compositions containing $Sn(NH_4)_2F_6$ is from 3.5 to 8.0. A 1000 ppm $F^-$ solution of $Sn(NH_4)_2F_6$ was stable. It did not become hazy on standing after several weeks. In contrast, a $SnF_2$ solution developed turbidity, probably due to oxy-fluoride formation, after 24 hours at room temperature.

$SnF_2$ at concentrations equivalent to 1000 ppm and 2500 ppm released, respectively, 80% and 45% of the available F. $Sn(NH_4)_2F_6$ displayed the same ionisation values as $SnF_2$. The corresponding values for $Na_2SnF_6$ are 73% and 38%, respectively. Thus $Na_2SnF_6$ is less ionised than $Sn(NH_4)_2F_6$.

$Sn(NH_4)_2F_6$ is significantly better tasting than $Na_2SnF_6$. A comparative test was carried out with a panel of 3 assessors, and tasting (20 sec. rinse with 10 ml of test solution containing 1000 ppm $F^-$) was performed with coded solutions or on a blind basis. The finding that $Sn(NH_4)_2F_6$ had a significantly less metallic taste than $Na_2SnF_6$ was the unanimous decision of the three judges. $Sn(NH_4)_2F_6$ was also much less astringent and metallic tasting than the uncomplexed fluoride salt $SnF_2$ at equal molar concentrations.

A similar blind test was conducted to compare the taste of $Sn(NH_4)_2F_6$ with that of $SnF_2$. This showed that $Sn(NH_4)_2F_6$ had a significantly less metallic taste than $SnF_2$ and this was the unanimous decision of the three judges.

It is generally agreed that oral bacteria are responsible for tooth decay. Therefore, a reduction of the cariogenic flora usually results in a decrease in caries formation. The inhibitory action of the test compounds was determined by adding each salt, at known concentrations, to a culture of cariogenic bacteria. After 24 hours at 37°C bacterial growth was monitored as a change in optical density. A dose response curve (concentration of salt vs. growth) for each compound against each bacterial isolate was plotted in order to obtain the concentration of test agent which would cause a 50% inhibition in growth (ID/50). This is a useful ranking index since it is not always possible to obtain complete inhibition with weak germicidal agents. Only those compounds which were able to elicit an ID/50 response were considered as inhibitory agents.

The medium used for bacterial growth experiments was Trypticase Soy Broth (B.B.L.: Baltimore Biological Labs., Baltimore, MD) supplemented with 0.1% Tween 80® (the word Tween is a trade mark) and 0.05% sodium thioglycollate. The test organisms, as lyophilised cells, were grown overnight in 50 ml of growth medium in a vacuum desiccator with an atmosphere of 97% $CO_2$/3% $H_2$ at 35°C. These cultures were then used at a 0.2% inoculum to prepare second stage seed flasks. The test media (10 ml) consisting of growth medium plus the potential inhibitors, contained in 16×120 mm screw top test tubes, were inoculated with the second stage seed cultures to given an absorbancy reading of 0.05. Immediately after inoculation the cultures were incubated for 24 hours under anaerobic conditions.

The growth medium was sterilised by autoclaving, whereas the potential inhibitors (as aqueous solutions) were sterilised by filtering each solution separately through a Millipore membrane (0.22 μm).

Absorbance at 600 nm versus a medium control was used as a measure of growth on a Beckman Model B spectrophotometer. All absorbancy readings were determined in 16 mm×120 mm Kimax test tubes. This instrument was linear to 0.60 OD units; thus, all cultures displaying values above this range were diluted with the control, accordingly.

TABLE I

Inhibition of bacterial growth

| | ID/50 (mM/L) | | | | | | |
|---|---|---|---|---|---|---|---|
| | S.mutans | | S.salivarius | | S.sanguis | | L.casei |
| Salts | 6715 | OMZ-176 | SS4 | H-257 | 10556 | 10557 | |
| * $SnCl_2$ | 2.26 | 3.68 | 1.45 | 3.64 | 1.27 | 1.88 | 3—6.0 |
| * $SnF_2$ | 1.50 | 2.28 | 2.45 | 0.74 | 0.44 | 0.84 | 2.97 |
| * $Sn(NH_4)_2F_6$ | 1.20 | 1.43 | 1.65 | 0.40 | 0.22 | 0.39 | 2.53 |
| * NaF | 7.3 | 16.30 | 13.40 | 3.77 | 1.18 | 1.82 | >12.00 |

The above values are the means of triplicate experiments.

The results show that $Sn(NH_4)_2F_6$ was more inhibitory than the non-fluoride salts and NaF, with no apparent changes in specificities. The ID/50 values, recalculated as ppm $F^-$, again show (Table II) that $Sn(NH_4)_2F_6$ is over-all more active than NaF.

TABLE II

Inhibition of bacterial growth by fluorine salts

| | ID/50 (ppm F) | | | | | | |
|---|---|---|---|---|---|---|---|
| | S.mutans | | S.salivarius | | S.sanguis | | L.casei |
| Salts | 6715 | OMZ-176 | SS4 | H-257 | 10556 | 10557 | |
| NaF | 138 | 309 | 255 | 72 | 22 | 35 | 352 |
| * $SnF_2$ | 56 | 87 | 93 | 29 | 17 | 32 | 113 |
| $Sn(NH_4)_2F_6$ | 137 | 163 | 188 | 46 | 25 | 45 | 288 |

It should be noted that $Sn(NH_4)_2F_6$, which is the stannic form of tin, was less active than the stannous form $SnF_2$.

Dental plaque is a film on the surface of teeth. This layer is the result of bacterial growth and is composed chiefly of microorganisms, proteinaceous materials and microbial by-products, such as glucans and organic acids. Plaque is not only primarily responsible for caries formation but it is also implicated in gingival diseases. Because the results described herein above have demonstrated that $Sn(NH_4)_2F_6$ possesses antibacterial activity, it is obvious that $Sn(NH_4)_2F_6$ will also reduce plaque formation.

The following are examples of a toothpaste and a mouthwash composition of the invention.

## 0 121 013

Example 1

The following is an example of a toothpaste composition according to the invention.

| | |
|---|---|
| Silica xerogel | 13.00 |
| Silica aerogel | 22.00 |
| Sorbitol (70% solution) | 41.50 |
| $Sn(NH_4)_2F_6$ | 0.24 |
| Sodium lauryl sulphate | 7.00 |
| Colour | 0.04 |
| Flavour | 3.00 |
| Sodium saccharin | 0.12 |
| Sodium benzoate | 0.06 |
| Titanium dioxide | 0.35 |
| Glycerine | 7.53 |
| Water | Balance to 100% |

Example 2

The following is an example of a mouthwash composition according to the present invention.

| | |
|---|---|
| Ethanol | 15.00 |
| Propylene glycol | 10.00 |
| Glycerol | 12.00 |
| Flavour, colour | 0.90 |
| Polyoxyethylene (20) monolaurate | 2.10 |
| Sodium lauryl sulphate | 0.33 |
| $Sn(NH_4)_2F_6$ | 0.024 |
| Buffer | 18.00 |
| Hydrochloric acid (to pH 4.1) | qs |
| Water | Balance to 100% |

Although the examples herein show the use of $Sn(NH_4)_2F_6$ in dentifrice and mouthwash preparations, the compounds of the present invention may also be incorporated in other oral preparations or formulations such as for example chewing gums and lozenges.

**Claims**

1. An oral composition having anti-caries and anti-plaque activity comprising 0.01 to 3% by weight of stannic ammonium fluoride [$Sn(NH_4)_2F_6$].

2. An oral composition as claimed in Claim 1, wherein the amount of $Sn(NH_4)_2F_6$ is sufficient to provide 1000 to 1500 ppm of available fluoride ion.

3. An oral composition as claimed in Claim 1 or Claim 2 in the form of a toothpaste or mouthwash.

**Patentansprüche**

1. Ein orales Präparat mit Antikaries- und Antiplaque-Wirksamkeit, dadurch gekennzeichnet, dass es 0.01 bis 3 Gew.% Zinnammoniumfluorid [$Sn(NH_4)_2F_6$] enthält.

2. Ein orales Präparat wie in Anspruch 1 beansprucht, dadurch gekennzeichnet, dass der Gehalt an $Sn(NH_4)_2F_6$ ausreichend ist, um 1000 bis 1500 ppm verfügbares Fluoridion zur Verfügung zu stellen.

3. Ein orales Präparat wie in Anspruch 1 oder Anspruch 2 beansprucht in Form einer Zahnpaste oder eines Mundwassers.

**Revendications**

1. Composition buccale ayant une activité anticaries et antiplaque, qui comprend 0,01 à 3% en poids de fluorure d'ammonium stannique $[Sn(NH_4)_2F_6]$.

2. Composition buccale selon la revendication 1, dans laquelle la quantité de $Sn(NH_4)_2F_6$ est suffisante pour fournir 1000 à 1500 ppm d'ions fluorure disponible.

3. Composition buccale selon la revendication 1 ou 2, sous forme d'une pâte dentifrice ou d'un bain de bouche.